# EUROPÄISCHE PATENTSCHRIFT

(11) **EP 3 068 314 B1**
(45) Veröffentlichungstag und Bekanntmachung des Hinweises auf die Patenterteilung: **08.04.2020**
(21) Anmeldenummer: 14721865.5
(22) Anmeldetag: 06.05.2014
(51) Int. Cl.: A61B 17/132, A61B 17/00, A61B 90/00

(54) **VENENSTAUER**
VENOUS TOURNIQUET
GARROT POUR VEINES

(30) Priorität: 15.11.2013 DE 102013112597
(43) Veröffentlichungstag der Anmeldung: 21.09.2016
(73) Patentinhaber: Kimetec GmbH, 71254 Ditzingen (DE)
(72) Erfinder: IHLE, Caroline, 71706 Markgröningen (DE); KIRCHNER, Claudia, 71706 Markgröningen (DE); KIRCHNER, Hansjörg, 71706 Markgröningen (DE)
(74) Vertreter: Fleck, Hermann-Josef
(86) Internationale Anmeldenummer: PCT/EP2014/059199
(87) Internationale Veröffentlichungsnummer: WO 2015/070992

(56) Entgegenhaltungen:
- WO-A2-2008/076820
- DE-A1-102009 025 416
- DE-U1-202013 100 312
- US-A- 5 036 838
- US-A1- 2005 131 322
- US-A1- 2012 122 365
- US-A1- 2012 310 273

## Beschreibung

Die Erfindung bezieht sich auf einen Venenstauer, mit einem um ein Körperteil legbaren Abschnürband, welches selbst in Umfangsrichtung elastisch ausgebildet ist oder mit mindestens einem elastischen Abschnitt versehen ist, und mit einer Verschlussvorrichtung, mittels deren das Abschnürband in seinem um ein Körperteil gelegten gespannten Zustand zu einer Spannschlaufe verriegelbar ist.

Ein Venenstauer dieser Art ist in der US 2012/0310273 A1 angegeben. Dieser bekannte Venenstauer weist ein Abschnürband mit einer glatten Seite und einer flauschigen Seite auf, wobei das Band mit in Längsrichtung parallel zueinander verlaufenden elastischen Fäden und in Querrichtung parallel zueinander und rechtwinklig zu den Längsfäden verlaufenden Fäden ausgebildet ist. Um die Längsfäden sind schleifenförmige Filamentfäden gelegt. Die flauschige Seite bildet Schlaufen für einen Klettverschluss und auf der glatten Seite sind Abschnitte mit Hakenelementen angebracht.

Ein derartiger Venenstauer ist in der US 3,930 506 offenbart. An einem Endabschnitt des Abschnürbandes ist zum Fixieren einer um das Körperteil unter Zugspannung gelegten Spannschlaufe ein Klebeabschnitt angebracht. Wird dieser nicht bei der ersten Fixierung in der richtigen Lage fixiert, ist eine zuverlässige nochmalige Fixierung nicht sichergestellt, wodurch sich Nachteile für die Benutzung ergeben können. Auch die Anbringung der Klebemittel ist mit entsprechendem Aufwand verbunden.

In der US 3,628,536 ist ein weiterer Venenstauer angegeben. Dieser weist eine Verschlussvorrichtung mit über die Länge verteilten Öffnungen und einem an einem Ende angebrachten Vorsprung auf, der zum Eingriff an die Öffnungen angepasst ist.

In der DE 20 2013 100 312 U1 ist ein Venenstauer mit einem Spannungsindikator gezeigt, der als ein einen elastischen Abschnitt an einem Abschnürband zwischen zwei Fixierstellen mit definierter Überlänge bezüglich des unbelasteten Abschnürbands überbrückender Bahnabschnitt aus einem flexiblen unelastischem Material ausgebildet ist. Außerdem ist eine Verschlussvorrichtung mit einem Halteteil genannt, welches als Klettverschlussabschnitt ausgebildet ist.

Ein weiterer Venenstauer ist in der US 3,930 506 offenbart. An einem Endabschnitt des Abschnürbandes ist zum Fixieren einer um das Körperteil unter Zugspannung gelegten Spannschlaufe ein Klebeabschnitt angebracht. Wird dieser nicht bei der ersten Fixierung in der richtigen Lage fixiert, ist eine zuverlässige nochmalige Fixierung nicht sichergestellt, wodurch sich Nachteile für die Benutzung ergeben können. Auch die Anbringung der Klebemittel ist mit entsprechendem Aufwand verbunden.

In der US 3,628,536 ist ein weiterer Venenstauer angegeben. Dieser weist eine Verschlussvorrichtung mit über die Länge verteilten Öffnungen und einem an einem Ende angebrachten Vorsprung auf, der zum Eingriff an die Öffnungen angepasst ist.

In der US 2012/0071917 A1 ist eine Abschnürvorrichtung für Körperteile zum Unterbinden eines starken Blutverlustes bei Verletzungen angegeben. Bei dieser bekannten Abschnürvorrichtung sind eine Spannvorrichtung zum Erzielen einer hohen Spannung des um das Körperteil zu einer Schlaufe gelegten Abschnürbandes sowie ein Spannungsindikator vorgesehen, mit dem eine erzeugte Spannung des Abschnürbandes taktil oder visuell festgestellt werden kann. Beispielsweise ist hierzu, wie in den dortigen Fig. 14A und 14B mit Beschreibung gezeigt, ein Spannungsgewebe gefaltet und im Faltenbereich an Befestigungsstellen festgelegt, die bei übermäßiger Spannung reißen. Die Befestigungsstellen können über die Breite des Spannungsgewebes unterschiedlich viele Befestigungspunkte aufweisen, wodurch die Befestigungsstellen bei entsprechend unterschiedlicher Zugspannung reißen. Allerdings muss eine derart hohe Zugspannung erst aufgebracht werden, um ein Reißen an der oder den Befestigungsstellen zu bewirken, wonach dann aber die Zugspannung abrupt nachlässt. Mit diesen Maßnahmen ist eine dosierte Spannung, wie sie beispielsweise bei Venenstauern zur Wirkung kommt, um einen venösen Blutfluss zu stauen, schwer erreichbar und erkennbar. Auch ist die Verschlussvorrichtung relativ aufwändig.

Weitere Abschnürvorrichtungen für Körperteile zeigen die US 5,653,728 A, die WO 2006/015987 A1, die GB 2 424 189 A, die US 6,525,238 B2, die US 2008/0312682 A1 sowie die US 6,250,047 B1. Die Abschnürvorrichtungen sind mit unterschiedlichen Verschlussvorrichtungen versehen, wobei die Verriegelung bzw. Fixierung zum Teil umständlich oder der Aufbau aufwändig ist. Dabei sind beispielsweise in der US 5,653,728 A und der WO 2006/015987 A1 auch als nach Gebrauch wegwerfbare Ausführungen von Abschnürvorrichtungen offenbart, wie sie häufig im klinischen Bereich zum Einhalten von Hygienevorschriften benötigt werden.

Die DE 10 2009 025 416 B4 offenbart einen Stütz- oder Fixiergurt mit zumindest einem flexiblen, unelastischen Abschnitt und Fixiereinrichtungen zum Festlegen von Gurtbereichen aneinander oder an orthopädietechnischen Einrichtungen. Die Fixiereinrichtungen können als Klettverschlusselemente ausgebildet und aufgenäht, aufgeklebt oder aufgeschweißt oder an Enden eines Gurtbandes angenäht sein. Auch kann die Oberseite eines durchgehenden elastischen Gurtabschnitts mit einer Flauschschicht ausgestattet sein, während an der Unterseite Fixiereinrichtungen zum Festlegen der Endbereiche aneinander oder an den orthopädietechnischen Einrichtungen vorgesehen sind. Ein solcher Stütz- oder Fixiergurt mit Fixiereinrichtungen in seinen Endbereichen ist nicht auf variable Schlaufengrößen einstellbar und zur Verwendung als Venenstauer nicht nutzbar.

Weitere bandartige Gurte oder Gurtabschnitte mit Klettverschluss sind in der EP 2 045 047 A2, der GB 479 442 A und der GB 1 033 130 A gezeigt.

Der Erfindung liegt die Aufgabe zugrunde, einen Venenstauer der eingangs genannten Art bereit zu stellen, der einen einfachen Aufbau mit sicherer Funktion besitzt und mit dem die Benutzung erleichtert wird.

Diese Aufgabe wird mit den Merkmalen des Anspruches 1 gelöst. Hierbei ist vorgesehen, dass die Verschlussvorrichtung als Klettverschluss mit zwei verriegelnd ineinander greifenden Verschlussteilen ausgebildet ist. Der Aufbau ergibt eine einfache Handhabung mit wenigen Handgriffen beim Festlegen an dem Körperteil, insbesondere Arm, mit genügender Zugspannung für das Stauen einer Vene. Vorteilhaft lässt sich die Abschnürvorrichtung für den Einmalgebrauch verwenden, um Hygieneanforderungen zu erfüllen. Die beiden Verschlussteile, mit Hakenelementen auf der einen Seite und Schlaufen auf der anderen Seite, ergeben eine stabile Verbindung mit hoher Zugfestigkeit in Flächen- bzw. Umfangsrichtung des Körperteils. Ein besonders vorteilhafter Aufbau für die Herstellung und einen einfachen Gebrauch wird dadurch erhalten, dass ein erster Verschlussteil aus dem Bandmaterial des Abschnürbandes selbst und ein zweiter Verschlussteil aus einem auf dem Bandmaterial befestigten Materialabschnitt gebildet ist oder in das Bandmaterial integriert ist.

Eine für die Funktion und die Anwendung vorteilhafte Ausbildung besteht darin, dass das Abschnürband aus flauschigem, reißfestem Material hoher Zugfestigkeit hergestellt ist.

Eine weitere vorteilhafte Ausgestaltung ergibt sich dadurch, dass das Abschnürband aus einem Vlies, wie Spinnvlies, oder aus Filz oder papierartigem Material mit aufgerauhter bzw. feinen Härchen aufweisender Oberfläche hergestellt ist. Das Vlies kann z. B. unter Wasserstrahlverfestigung hergestellt sein.

Bei der erfindungsgemäßen Ausgestaltung ist das Abschnürband aus einem Spinnvlies hergestellt.

Verschiedene weitere vorteilhafte Ausgestaltungsvarianten werden dadurch erhalten, dass das Abschnürband aus Polyester, Polypropylen und/oder Viskosematerial hergestellt ist.

Für die Funktion und Bedienung ist weiterhin vorteilhaft, dass das Abschnürband und der zweite Verschlussteil gleiche Breite im Bereich von 1 cm bis 4 cm haben, vorzugsweise zwischen 1,5 cm und 3 cm.

Für den Gebrauch ergeben sich weitere Vorteile dadurch, dass mindestens ein Spannungsindikator zum Feststellen eines Spannungszustandes des Abschnürbandes vorhanden ist, der in der Weise ausgebildet ist, dass ein definierter Spannungszustand von einem Benutzer erkennbar ist.

Mit diesen Maßnahmen kann der Benutzer, bei der als Venenstauer ausgebildeten Abschnürvorrichtung, die zum Unterbinden des Blutflusses erforderliche, relativ geringe Zugspannung (von z. B. ca. 10 mm Hg im Bereich von Kapillargefäßen oder < 25 mm Hg im Bereich größerer Venen) exakt einstellen. Hierdurch wird auch die behandelte Person nicht mehr als nötig belastet.

Eine erfindungsgemäße Ausgestaltung zum Erkennen der erzeugten Zugspannung besteht darin, dass der Spannungsindikator als ein einen elastischen Abschnitt am Abschnürband in Dehnrichtung zwischen zwei Fixierstellen mit definierter Überlänge bezüglich des unbelasteten Abschnürbandes überbrückender Bahnabschnitt aus einem flexiblen unelastischen Material ausgebildet ist. Ist der mit einer Überlänge bezüglich des Abstandes der Fixierstellen im spannungslosen Zustand des Abschnürbandes (z. B. als gefalteter Abschnitt) ausgeführte Bahnabschnitt noch locker, so ist ein definiert vorgegebener Spannungszustand noch nicht erreicht. Ist hingegen der flexible unelastische Bahnabschnitt infolge des gedehnten elastischen Abschnitts zwischen den Fixierstellen vollkommen entlang dem Abschnürband erstreckt, so ist der definierte Spannungszustand zum Unterdrücken des venösen Blutflusses erreicht. Durch die Überlänge des Bahnabschnitts zwischen den beiden Fixierstellen kann somit ein gewünschter definierter Spannungszustand für einen jeweiligen Anwendungsfall herstellerseitig vorgegeben werden, der bei der Anwendung von dem Benutzer leicht und eindeutig erkennbar und einstellbar ist.

Eine vorteilhafte Ausgestaltungsvariante besteht dabei darin, dass das Abschnürband aus einem flexiblen unelastischen Material ausgebildet ist und der Bahnabschnitt einen integralen Teil des Abschnürbandes bildet, wobei an den Fixierstellen ein den elastischen Abschnitt bildendes elastisches Element angebunden ist.

Eine andere vorteilhafte Ausgestaltungsvariante besteht darin, dass das Abschnürband selbst zumindest abschnittsweise als elastisches Band ausgebildet ist und der Bahnabschnitt aus unelastischem Material an den Fixierstellen an dem elastischen Band angebunden ist.

Eine für einen Einmalgebrauch vorteilhafte Abschnürvorrichtung besteht darin, dass der elastische Abschnitt oder ein anderer Abschnitt des elastischen Bandes mit einem bei einer ersten Dehnung irreversibel durchreißenden oder beschädigten Gebrauchsindikator versehen ist, wie z B. aus Papier oder Lack. Die Durchreißkraft des Gebrauchsindikators ist hierbei vorteilhaft gering, so dass sie den Spannungsvorgang des Abschnürbandes möglichst wenig beeinflusst und der Durchriss beispielsweise bereits bei geringer Dehnung von wenigen Prozent der Dehnung bei dem definierten Spannungszustand (z. B. nicht mehr als 10 % oder 20 % der Dehnung im definierten Spannungszustand) eintritt. Dies kann durch z. B. entsprechende Materialwahl und/oder Materialstärke und/oder Form des Gebrauchsindikators oder durch Abriss an einer Anbindungsstelle erreicht werden.

Für die Fertigung und Funktion sind die Maßnahmen von Vorteil, dass die Anbindung durch Befestigen des zweiten Verschlussteils und gegebenenfalls des elastischen Abschnitts oder des Bahnabschnitts an dem Abschnürband an den Fixierstellen mittels Klebens, Schweißens, thermoplastischer Verfahren oder Vernähens hergestellt ist.

Die Erfindung wird nachfolgend anhand von Ausführungsbeispielen unter Bezugnahme auf die Zeichnungen näher erläutert. Es zeigen:
- Fig. 1A und 1B: ein erstes Ausführungsbeispiel für eine Abschnürvorrichtung in zwei verschiedenen perspektivischen Ansichten,
- Fig. 2: die Abschnürvorrichtung nach Fig. 1A in Draufsicht, in seitlicher Ansicht und in einer Ansicht von unten,
- Fig. 3A und 3B: ein weiteres Ausführungsbeispiel der Abschnürvorrichtung im nicht gespannten Zustand des Abschnürbandes und im gespannten Zustand desselben in Draufsicht, Seitenansicht, Unteransicht und perspektivischer Ansicht sowie vergrößerte Ausschnittsdarstellungen in den beiden Zuständen und
- Fig. 4A und 4B: ein weiteres Ausführungsbeispiel für eine Abschnürvorrichtung im nicht gespannten und im gespannten Zustand in Seitenansicht und Unteransicht.

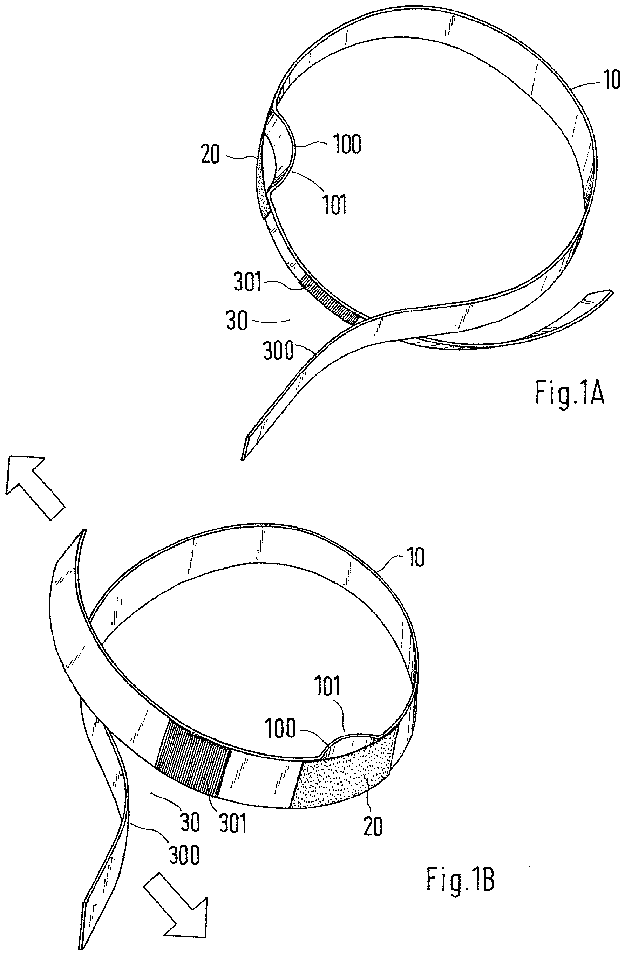

Fig. 1A und 1B zeigen ein erstes Ausführungsbeispiel für eine als Venenstauer ausgebildete Abschnürvorrichtung 1 mit einem Abschnürband 10, das zum zumindest teilweisen Unterbinden eines Blutflusses in Form einer geschlossenen Spannschlaufe um einen Körperteil legbar und imgespannten Zustand mittels einer Verschlussvorrichtung 30 fixierbar ist. Die Verschlussvorrichtung 30 ist als Klettverschluss mit zwei verriegelnd ineinander greifenden Verschlussteilen, nämlich einem ersten Verschlussteil 300 und einem zweiten Verschlussteil 301 ausgebildet.

Die Abschnürvorrichtung 1 weist bei dem gezeigten Ausführungsbeispiel einen Spannungsindikator 101 auf, mit dem die Spannkraft des Abschnürbandes 10 im Bereich der um einen Körperteil gelegten Spannschlaufe von einem Benutzer erkennbar ist. Der Spannungsindikator 101 ist vorzugsweise in der Nähe des verschlussseitigen Bandabschnitts angeordnet, so dass er in jedem Falle der durch die Spannschlaufe an dem angelegten Körperteil ausgeübten Spannkraft unterliegt.

Bei dem in den Fig. 1A und 1B gezeigten Ausführungsbeispiel ist das Abschnürband 10 selbst als erstes Verschlussteil 300 der Verschlussvorrichtung 30 ausgebildet, während der zweite Verschlussteil 301 als auf dem Abschnürband 10 befestigter Materialabschnitt ausgebildet ist. Eine erfindungsgemäße Ausgestaltung besteht dabei darin, dass das Abschnürband 10 aus einem flauschigen, bezüglich der auftretenden Dehnkräfte genügend reißfesten bzw. genügend hohe Zugfestigkeit aufweisenden Material hergestellt ist, welches Schlaufen (Schlingen) für die Klettverriegelung bildet, während der zweite Verschlussteil 301 als kurzer Abschnitt aus Flachmaterial mit an die Schlaufen des ersten Verschlussteils 300 angepassten Hakenelementen ausgeführt ist.

Die Hakenelemente weisen vorzugsweise der Zugkraft bzw. Spannkraft des um das betreffende Körperteil gelegten Bandabschnitts entgegen gerichtete Häkchen auf; sie können aber auch als Pilzköpfe, schuppenartige Elemente oder dgl. ausgebildet sein, wobei die Schlaufen bzw. das diese enthaltende Schlinggewebe entsprechend angepasst sind. Die Schlaufen, die nicht notwendigerweise als geschlossene Schlaufen ausgebildet sein müssen, und die Hakenelemente können insbesondere in miniaturisierter Ausbildung sich in einer Vielzahl von feinen Härchen nach Art eines Gecko-Effektes ineinander verkrallen. Vorteilhaft sind die Schlaufen und Hakenelemente der beiden Verschlussteile als Mikroelemente in der Weise ausgebildet, dass sich das Abschnürband auch bei direktem Kontakt mit der Haut eines abzuschnürenden Körperteils flauschig anfühlt.

In Untersuchungen hat sich gezeigt, dass der in genannter Weise ausgebildete Klettverschluss im angelegten Zustand genügend hohe Zugkräfte (Scherkräfte) parallel zur Fläche bzw. in Umfangsrichtung des Abschnürbands 10 aufnimmt, um die erforderliche Abschnürung z. B. von Venen zu gewährleisten, wobei ein Spannungszustand des Abschnürbandes 10 zum Erzeugen eines Drucks von z. B. bis zu 10 mm Hg oder bis zu 25 mm Hg im Bereich der Venen zuverlässig erreicht werden kann. Zum Anlegen und Abnehmen lässt sich der Klettverschluss leicht und schnell schließen bzw. öffnen.

Das z. B. zwischen 1 und 5 cm (vorzugsweise zwischen 1,5 cm und 3 cm) breite Abschnürband 10 kann aus sehr dünnem (weniger als 1 mm) Rollen- oder Flächenmaterial geschnitten werden und ist sehr flexibel bei hoher Reißfestigkeit. Es kann selbst eine z. B. geringe Eigenelastizität aufweisen, so dass es nach Anlegen an dem Körperteil selbst eine elastische Spannkraft erzeugt. Alternativ oder zusätzlich kann die Spannkraft durch ein aus geeignetem Material hergestelltes elastisches Element 20 erzeugt werden, das im Bereich des Spannungsindikators 101 oder außerhalb desselben an dem Abschnürband 10 befestigt ist.

Der zweite Verschlussteil 301 besitzt vorteilhaft die gleiche Breite wie das Abschnürband 10 und vorzugsweise eine geringe Länge z. B. zwischen 1 cm und 5 cm. Er kann sich alternativ auch über die gesamte oder nahezu gesamte Länge des Abschnürbandes 10 erstrecken. Der zweite Verschlussteil 301 ist seinerseits aus dünnem, flexiblem Material hergestellt und z. B. durch Schweißen (Laserschweißen, Ultraschallschweißen oder dgl.), Kleben, thermoplastische Verbindungsverfahren, Annähen oder dgl. in einem Endabschnitt des Abschnürbands 10 bzw. über die gesamte oder nahezu gesamte Länge desselben befestigt. Zum Verriegeln bzw. Schließen des Klettverschlusses braucht nur der um das Körperteil gelegte andere Schlaufenabschnitt auf den Verschlussteil 301 angedrückt zu werden, womit sich die Verschlussteile ineinander verriegeln. Eine weitere Ausführungsvariante besteht darin, dass Schlaufen (bzw. Schlingen) in derselben Schicht des Vliesstoffes bzw. Schlingengewebes ineinander integriert sind.

Geeignete Materialien für das Abschnürband bzw. den Klettverschluss sind Viskose, Polyester, Polypropylen, wobei erfindungsgemäß Spinnvlies in hautfreundlicher Ausführung verwendet wird.

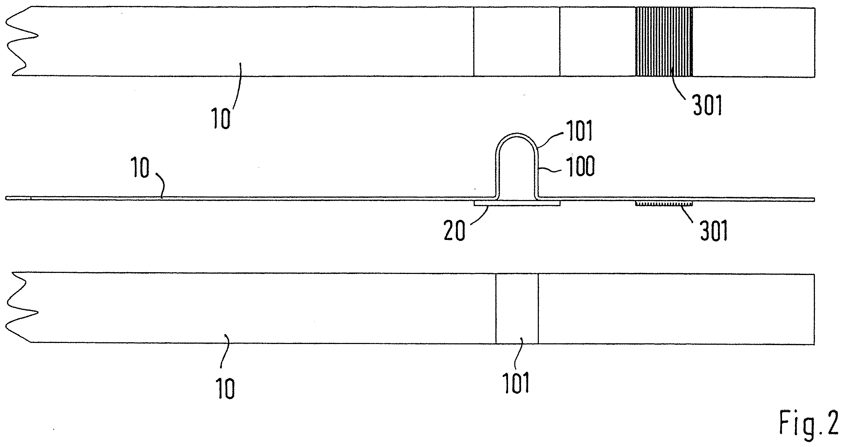

Fig. 2 zeigt einen Abschnitt der als Venenstauer ausgebildeten Abschnürvorrichtung mit dem Abschnürband 10, einem Spannungsindikator 101 mit einem Dehnabschnitt 100 und einem überbrückenden elastischen Element sowie einem zweiten Verschlussteil 301 des Klettverschlusses in Draufsicht, seitlicher Ansicht und Unteransicht.

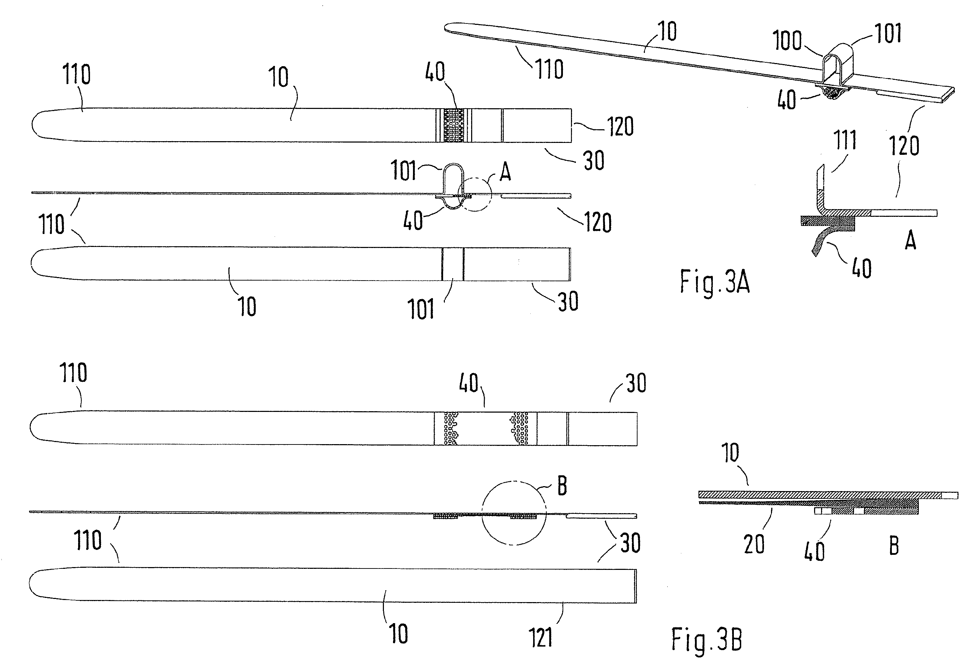

In Fig. 3A ist die als Venenstauer ausgebildete Abschnürvorrichtung 1 im nicht gespannten Zustand des Abschnürbandes 10 in Draufsicht, in Seitenansicht und in Unteransicht dargestellt. In Fig. 3B ist die Abschnürvorrichtung bzw. der Venenstauer in einem gespannten Zustand des Abschnürbandes 10 ebenfalls mit einem vergrößerten Ausschnitt B im Bereich des Spannungsindikators 101 wiedergegeben, wobei das Abschnürband 10 im Bereich eines Dehnabschnitts 100, in dem ein elastisches Element 20 angeordnet ist, um eine definierte Weglänge gedehnt ist. Sobald diese definierte Wegstrecke ausgehend von dem ungespannten Zustand des Abschnürbandes 10 bzw. einem weniger gespannten Zustand erreicht ist, liegt ein definierter Spannungszustand des Abschnürbandes 10 vor. Die dabei bewirkte Spannkraft kann durch die Ausbildung des Dehnabschnitts 100, z. B. durch eine betreffende Materialwahl (Materialeigenschaften und/oder geometrische Eigenschaften, wie Länge, Dicke, Breite, Aussparungen oder dgl.) bei der Fertigung definiert eingestellt werden. In den Fig. 3A und 3B ist auch ein weiter unten noch näher beschriebener Gebrauchsindikator 40 gezeigt.

In dem gezeigten Ausführungsbeispiel kann das Abschnürband 10 selbst aus inelastischem, aber flexiblem Material hergestellt sein. Es kann bei der Fertigung der Abschnürvorrichtung 1 als Rollenware vorgehalten und entsprechend der gewünschten Länge des Abschnürbandes 10 abgelängt werden. Vorteilhaft ist ein recyclefähiges Material, wobei auch z. B. natürliche Verstärkungsfasern aus nachwachsenden Rohstoffen eingebunden und auch die übrigen Teile, wie Verschlussvorrichtung, Dehnabschnitt, Befestigungsmittel und dgl. unter Berücksichtigung einer guten Recyclefähigkeit ausgewählt werden können. Die Herstellung, Funktionsweise und die Gestaltung der Abschnürvorrichtung 1 sind vorteilhaft auch hinsichtlich einer nur einmaligen Verwendung auslegbar.

Der Spannungsindikator 101 ist bei dem in Fig. 1A und 1B gezeigten Ausführungsbeispiel z. B. als Teil eines im Wesentlichen inelastischen Abschnürbandes 10 selbst ausgebildet, indem es in Überlänge an zwei in Dehnrichtung voneinander beabstandeten Fixierstellen an dem aus elastischem Material bestehenden Dehnabschnitt 100 angebunden ist. Zwischen den beiden Fixierstellen verläuft das dort den Spannungsindikator 101 bildende Abschnürband 10 faltenförmig, wobei die Überlänge auf den gewünschten, zu erzeugenden definierten Spannungszustand auch in Abhängigkeit von der Ausbildung des Dehnabschnitts 100 abgestimmt ist. Die Anbindung an den Fixierstellen erfolgt genügend fest z. B. durch Kleben, Schweißen (Laserschweißen, Ultraschweißen oder dgl.), thermoplastische Verbindungsverfahren, Annähen oder dgl.. Beim Spannen des Abschnürbandes 10 kann der Benutzer anhand der sich mehr und mehr dem Dehnabschnitt 100 annähernden Falte den Dehnvorgang und damit die sich erhöhende Spannkraft kontrollieren und eindeutig erkennen, wenn der die beiden Fixierstellen überbrückende Bahnabschnitt des Abschnürbandes 10 seine maximale Strecklage erreicht hat. In diesem Moment ist auch der eingestellte, definierte Spannungszustand bzw. die dabei erzeugte optimale Spannkraft des Abschnürbands 10 erreicht. Soll z. B. der venöse Blutfluss im Bereich der Kapillaren im Übergangsbereich zwischen den Arteriolen und Venolen unterbunden werden, reicht ein dementsprechend eingestellter, definierter Spannungszustand von ca. 10 mm Hg, wobei die zu behandelnde Person wenig belastet wird. Im Bereich von Venen kann der Blutfluss bzw. die Pulsation z. B. durch einen Spannungszustand des Abschnürbandes 10 entsprechend einem Druck von kleiner als 25 mm Hg erreicht werden. Hierfür oder für andere Anwendungen können mit den vorstehend genannten Maßnahmen entsprechend ausgebildete Abschnürvorrichtungen 1 zur Verfügung gestellt werden, wobei der Spannungsindikator 101 und der Dehnabschnitt 100 exakt auf den jeweiligen Anwendungsfall abgestimmt sind.

In entsprechender Weise können auch mehr als ein Spannungsindikator vorzugsweise mit unterschiedlich definierten Spannungszuständen angebracht werden.

Bei einem anderen Ausführungsbeispiel der Abschnürvorrichtung 1 kann das Abschnürband 10 selbst aus elastischem Material ausgeführt sein. Ist daran an in Dehnungsrichtung voneinander beabstandeten Fixierstellen ein Spannungsindikator 101 mit definierter Überlänge angebunden, so besteht dieser vorteilhaft aus flexiblem unelastischem Material, wie bei dem vorhergehenden Ausführungsbeispiel. Wird das Abschnürband 10 gedehnt, legt sich der gefaltete Bahnabschnitt des Spannungsindikators 101 mehr und mehr an die außenseitige Oberfläche des Abschnürbandes 10 an. Dabei ist die Überlänge des Bahnabschnittes auf den überbrückten Abschnitt des elastischen Abschnürbandes abgestimmt, der dann dem elastischen Element 20 nach den vorherigen Ausführungsbeispielen entspricht, und zwar in der Weise, dass bei vollständiger Erstreckung des Bahnabschnitts entlang dem betreffenden Abschnitt des Abschnürbandes 10 ohne Falte der gewünschte definierte Spannungszustand erreicht ist. In diesem Zustand erfolgt dann die Fixierung der Schlaufe durch den Benutzer mittels des Klettverschlusses, ohne das Abschnürband noch stärker zu spannen.

Eine weitere Ausgestaltung der Abschnürvorrichtung 1 ist in Fig. 3A und 3B gezeigt. Hierbei ist die Abschnürvorrichtung entsprechend dem Ausführungsbeispiel nach Fig. 1A mit einem Spannungsindikator 101 versehen, der als Bahnabschnitt in Überlänge bezüglich des unbelasteten Abschnürbandes ein elastisches Element 20 zwischen zwei Fixierstellen überbrückt, wie vorstehend näher beschrieben. Auch die Verschlussvorrichtung 30 ist entsprechend dem Ausführungsbeispiel nach Fig. 1A ausgebildet. Zusätzlich ist jedoch ein Gebrauchsindikator 40 vorhanden, der bei dem gezeigten Ausführungsbeispiel ebenfalls das elastische Element 20 oder zumindest einen in Längsrichtung erstreckten Abschnitt desselben überbrückt.

Der Gebrauchsindikator 40 ist als in Längsrichtung bzw. Dehnrichtung sich erstreckendes Durchreißelement ausgebildet, welches bei Dehnung des elastischen Elements 20 mit geringem Kraftaufwand reißt, bevor die endgültige Dehnung erreicht ist, die bei dem definierten Spannungszustand vorliegt. Ähnlich dem Durchreißelement kann auch ein anderes irreversibel zu schädigendes Element, wie z. B. ein Lack, angebracht werden. In welchem Bereich während des Dehnvorgangs das Durchreißelement reißen soll, kann ebenfalls durch eine gewisse Überlänge zwischen zwei Fixierungsstellen bzw. Befestigungsstellen des Durchreißelements an dem elastischen Element 20 bestimmt werden, wobei die Überlänge des Durchreißelements aber jedenfalls geringer ist als die Überlänge des für den Spannungsindikator 101 vorgesehenen Bahnabschnitts. Beispielsweise kann auf diese Weise bestimmt werden, dass das Durchreißelement bereits durchreißt, wenn die Dehnung des elastischen Elements z. B. ca. 20 %, ca. 50 % oder ca. 80 % oder einen anderen, geringeren Zwischenwert relativ zu der bei dem definierten Spannungszustand vorliegenden Dehnung erreicht hat. Beliebige Zwischenwerte für ein Durchreißen können auf diese Weise exakt eingestellt werden. Ist das Durchreißelement des Gebrauchsindikators 40 gerissen, bedeutet dies, dass die Abschnürvorrichtung 1 bereits in Gebrauch war und somit keine Erstverwendung vorliegt, wie es bei Abschnürvorrichtungen 1 für den Einmalgebrauch erforderlich ist. Das Durchreißelement kann z. B. aus einem leicht reißenden Papierstreifen oder Faserstreifen bestehen und ist vorzugsweise inelastisch, jedoch flexibel.

Sind die Fixierstellen des Spannungsindikators 101 und die Befestigungsstellen des Gebrauchsindikators 40 in Draufsicht deckungsgleich, so können beide Indikatoren einfach in einem gemeinsamen Arbeitsschritt hergestellt und auch als Funktionseinheit aufeinander exakt in gewünschter Weise abgestimmt werden.

Die Anbindung des für den Gebrauchsindikator 40 verwendeten Durchreißelements an den betreffenden Fixierstellen bzw. Befestigungsstellen kann in entsprechender Weise erfolgen, wie im Zusammenhang mit dem elastischen Element 20 beschrieben, also beispielsweise durch Kleben, Schweißen (Laserschweißen, Ultraschall, Aufdruck mittels Drucktechnik oder dgl.), Vernähen oder eine thermoplastische Verbindungstechnik oder aber eine andere geeignete Befestigungsart. In der vergrößerten Darstellung des Ausschnitts A ist ein Verbindungsabschnitt (Befestigungsstelle) gezeigt, wobei das elastische Element 20 auf seiner Oberseite an dem Abschnürband 10 fixiert ist und auf seiner Unterseite mit dem betreffenden Befestigungsabschnitt des Durchreißelements verbunden ist.

In der Fig. 3B ist der gedehnte Zustand des Abschnürbandes 10 gezeigt, wie er beispielsweise in dem definierten Spannungszustand vorliegt. Hierbei ist das Durchreißelement des Gebrauchsindikators 40 durch die Dehnung bereits gerissen, wobei die beiden rissseitigen Ränder um einen relativ großen Dehnspalt voneinander beabstandet sind, d. h. der Durchriss bereits in einem entsprechend frühen Stadium der Dehnung erfolgt ist. In der vergrößerten Darstellung des Durchrissbereichs (Detail B) sind der eine (verschlussseitige) verbleibende Abschnitt des Durchreißelements, das bei der Dehnung dünner werdende elastische Element 20 sowie der gestreckte Bahnabschnitt des Spannungsindikators 101 zu sehen.

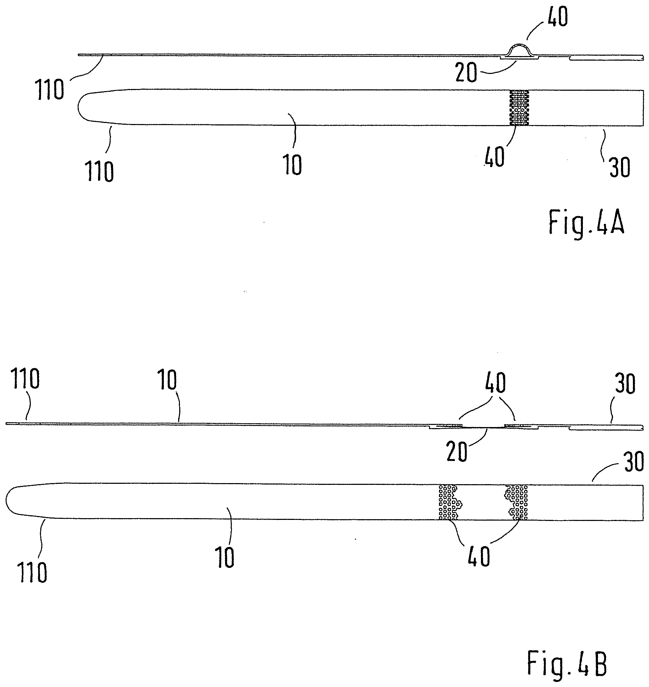

Wie Fig. 4A und 4B zeigen, kann die Abschnürvorrichtung 1 auch unabhängig von einem Spannungsindikator 101 oder davon in Längsrichtung versetzt einen Gebrauchsindikator 40 an dem Abschnürband 10 aufweisen, wobei letzterer ein elastisches Element 20 zwischen zwei Fixierstellen bzw. Befestigungsstellen mit (oder alternativ ohne) Überlänge überbrückt, ähnlich wie bei der Ausführung nach Fig. 3A, 3B beschrieben. Das Abschnürband 10 kann außerhalb des elastischen Elements 20 wiederum aus inelastischem (= unelastischem), flexiblem Material oder aus elastischem Material hergestellt sein.

## Patentansprüche

1. Venenstauer mit einem um ein Körperteil legbaren Abschnürband (10), welches selbst in Umfangsrichtung elastisch ausgebildet ist oder mit mindestens einem elastischen Abschnitt versehen ist, und mit einer Verschlussvorrichtung (30), mittels deren das Abschnürband (10) in seinem um ein Körperteil gelegten gespannten Zustand zu einer Spannschlaufe verriegelbar ist, wobei die Verschlussvorrichtung als Klettverschluss mit zwei verriegelnd ineinander greifenden Verschlussteilen ausgebildet ist, wobei ein erster Verschlussteil (300) aus dem Bandmaterial des Abschnürbandes (10) selbst und ein zweiter Verschlussteil (301) aus einem auf dem Bandmaterial befestigten Materialabschnitt gebildet ist und der erste Verschlussteil (300) mit Schlaufen und der zweite Verschlussteil (301) mit Hakenelementen versehen sind, die zum Verhaken mit den Schlaufen ausgebildet sind, und wobei das Abschnürband (10) aus flauschigem, reißfestem Material hoher Zugfestigkeit hergestellt ist,
wobei das flauschige reißfeste Material hoher Zugfestigkeit aus einem Spinnvlies hergestellt ist und
wobei ein Spannungsindikator (101) zum Feststellen eines Spannungszustandes des Abschnürbandes (10) vorhanden ist, der in der Weise ausgebildet ist, dass ein herstellerseitig definiert vorgegebener Spannungszustand von < 25 mm Hg zum Unterbinden eines venösen Blutflusses von einem Benutzer erkennbar ist, wobei der Spannungsindikator (101) als ein einen elastischen Abschnitt am Abschnürband (10) in Dehnrichtung zwischen zwei Fixierstellen mit definierter Überlänge bezüglich des unbelasteten Abschnürbandes (10) überbrückender Bahnabschnitt aus einem flexiblen unelastischen Material ausgebildet ist.

2. Venenstauer nach Anspruch 1,
**dadurch gekennzeichnet,**
**dass** das Abschnürband aus Polyester, Polypropylen und/oder Viskosematerial hergestellt ist.

3. Venenstauer nach einem der vorhergehenden Ansprüche,
**dadurch gekennzeichnet,**
**dass** das Abschnürband und der zweite Verschlussteil (301) gleiche Breite im Bereich von 1 cm bis 4 cm haben.

4. Venenstauer nach einem der vorhergehenden Ansprüche,
**dadurch gekennzeichnet,**
**dass** das Abschnürband (10) aus einem flexiblen unelastischen Material ausgebildet ist und der Bahnabschnitt einen integralen Teil des Abschnürbandes (10) bildet, wobei an den Fixierstellen ein den elastischen Abschnitt bildendes elastisches Element (20) angebunden ist.

5. Venenstauer nach einem der Ansprüche 1 bis 3,
**dadurch gekennzeichnet,**
**dass** das Abschnürband (10) selbst zumindest abschnittsweise als elastisches Band ausgebildet ist und der Bahnabschnitt aus unelastischem Material an den Fixierstellen an dem elastischen Band angebunden ist.

6. Venenstauer nach einem der vorhergehenden Ansprüche,
**dadurch gekennzeichnet,**
**dass** der elastische Abschnitt oder ein anderer Abschnitt des elastischen Bandes mit einem bei einer ersten Dehnung durchreißenden Gebrauchsindikator (40) versehen ist.

7. Venenstauer nach einem der vorherigen Ansprüche,
**dadurch gekennzeichnet,**
**dass** die Anbindung des zweiten Verschlussteils (301) und gegebenenfalls des elastischen Abschnitts oder des Bahnabschnitts an dem Abschnürband (10) an den Fixierstellen mittels Klebens, Schweißens, thermoplastischer Verfahren oder Vernähens hergestellt ist.

## Claims

1. Tourniquet with a constricting strap (10), which is placeable around a body part, which strap is itself elastic in the circumferential direction or is provided with at least one elastic section and with one closure device (30), by means of which the constricting strap (10), when in its tensioned state around the body part, is lockable to form a tensioning loop, wherein the closure device is designed as a hook and loop fastener with two interlocking fastening parts, wherein a first fastening part (300) is formed from the strap material of the constricting strap (10) itself and a second fastening part (301) is formed of a section of material fastened to the strap material, and, wherein the first fastening part (300) is provided with loops and the second fastening part (301) is provided with hook elements which are designed to interlock with the loops, and wherein the constricting strap (10) is made of fuzzy, tear-resistant material of high tensile strength and wherein the fuzzy, tear-resistant material of high tensile strength is produced from a spunbonded nonwoven and wherein a tension indicator (101), for determining the state of tension of the constricting strap (10), is provided, which indicator is designed in such a way that a manufacturer-defined prescribed state of tension of <25 mm Hg for preventing a venous blood flow is recognizable by a user, wherein the tension indicator (101) is designed as an elastic section bridging an elastic section on the constricting strap (10) between two fastening points in the stretching direction with a defined excess length with respect to the unstressed constricting strap (10) made of a flexible non-elastic material.

2. Tourniquet according to claim 1, **characterized in that** the constricting strap is made of polyester, polypropylene and/or viscose material.

3. Tourniquet according to one of the preceding claims, **characterized in that** the constricting strap and the second fastening part (301) have the same width in the range from 1 cm to 4 cm.

4. Tourniquet according to one of the preceding claims, **characterized in that** the constricting strap (10) is formed from a flexible, non-elastic material and wherein the web section forms an integral part of the constricting strap (10), with an elastic element forming the elastic section is connected at the fastening points (20).

5. Tourniquet according to one of claims 1 to 3, **characterized in that** the constricting strap (10) itself is formed, at least in sections, as an elastic strap and the web section, made of non-elastic material is connected at the fastening points to the elastic strap.

6. Tourniquet according to one of the preceding claims, **characterized in that** the elastic section or another section of the elastic strap is provided with a use indicator (40) that is torn through when first stretched.

7. Tourniquet according to one of the preceding claims, **characterized in that** the connection of the second fastening part (301) and optionally the elastic section or the web section to the constricting strap (10) is made at the fastening points by means of bonding, welding, thermoplastic processes or sewing.

## Revendications

1. Garrot comprenant une bande de serrage (10) pouvant être placée autour d'une partie du corps et qui est elle-même réalisée de façon élastique dans la direction périphérique ou est dotée d'au moins une partie élastique, et comprenant un dispositif de fermeture (30) au moyen duquel la bande de serrage (10) peut être bloquée à une boucle de serrage dans son état tendu autour d'une partie du corps, le dispositif de fermeture étant réalisé sous la forme d'une fermeture à Velcro comprenant deux parties de fermeture s'engrenant l'une dans l'autre de façon bloquante, une première partie de fermeture (300) étant constituée du matériau de la bande de serrage (10) elle-même et une seconde partie de fermeture (301) étant constituée d'une partie de matériau fixée au matériau de bande, et la première partie de fermeture (300) étant dotée de boucles et la seconde partie de fermeture (301) étant dotée d'éléments de crochet conçus pour s'accrocher aux boucles, et la bande de serrage (10) étant constituée d'un matériau pelucheux résistant à la déchirure et présentant une résistance élevée à la traction, le matériau pelucheux résistant à la déchirure et présentant une résistance élevée à la traction étant constitué d'un tissu filé-lié, et un indicateur de tension (101) étant prévu pour détecter un état de tension de la bande de serrage (10), lequel indicateur de tension est conçu de telle façon qu'un état de tension prédéfini par le fabricant de < 25 mm Hg pour empêcher un écoulement sanguin veineux d'un utilisateur puisse être détecté, l'indicateur de tension (101) étant réalisé sous la forme d'une partie de bande constituée d'un matériau flexible non élastique, qui ponte une partie élastique au niveau de la bande de serrage (10) dans la direction d'étirement entre deux points de fixation avec une surlongueur définie par rapport à la bande de serrage (10) non chargée.

2. Garrot selon la revendication 1, **caractérisé en ce que** la bande de serrage est fabriquée à partir du polyester, du polypropylène et/ou d'un matériau de viscose.

3. Garrot selon l'une des revendications précédentes, **caractérisé en ce que** la bande de serrage et la seconde partie de fermeture (301) ont la même largeur comprise entre 1 et 4 cm.

4. Garrot selon l'une des revendications précédentes, **caractérisé en ce que** la bande de serrage (10) est constituée d'un matériau non élastique flexible, et **en ce que** la partie de bande fait partie intégrante de la bande de serrage (10), un élément élastique (20) formant la partie élastique étant relié aux points de fixation.

5. Garrot selon l'une des revendications 1 à 3, **caractérisé en ce que** la bande de serrage (10) elle-même est au moins partiellement réalisée sous la forme de bande élastique, et **en ce que** la partie de bande en matériau non élastique est reliée aux points de fixation au niveau de la bande élastique.

6. Garrot selon l'une des revendications précédentes, **caractérisé en ce que** la partie élastique ou une autre partie de la bande élastique est équipée d'un indicateur d'utilisation (40) se rompant lors d'un premier étirement.

7. Garrot selon l'une des revendications précédentes, **caractérisé en ce que** la liaison de la seconde partie de fermeture (301) et éventuellement de la partie élastique ou de la partie de bande aux points de fixation au niveau de la bande de serrage (10) est réalisée par collage, soudage, par des procédés thermoplastiques ou par couture.
